# EUROPEAN PATENT APPLICATION

(11) **EP 3 719 082 A1**
(43) Date of publication of application: **07.10.2020**
(21) Application number: 19218859.7
(22) Date of filing: 20.12.2019
(51) Int. Cl.: C09C 1/00, A61K 8/19, A61Q 17/04, A61Q 19/00, C09D 7/48

(54) **A STABLE REVERSIBLE-COLOURED PHOTOCHROMIC ADDITIVE**

(30) Priority: 06.04.2019 IN 201921013941; 31.07.2019 WO PCT/IN2019/050565
(71) Applicant: Koel Colours Pvt. Ltd., 400 072 Mumbai (IN)
(72) Inventor: DESAI, Dhirubhai Bhikhubhai, 400 072 Mumbai (IN)
(74) Representative: Barker Brettell LLP

(57) **Abstract**

A stable and consistent reversible-coloured photochromic additive changes colour from an original buff colour to a desired colour shade, between pink to blue, when exposed to sunlight and restores to the original colour in the absence of the sunlight. It may simultaneously have an SPF value of at least 3. The additive comprises titanium dioxide, psuedoboehmite, iron oxide hydroxide and tungsten oxide.

## Description

The present invention relates in general to photochromic additives, particularly to a stable reversible-coloured photochromic additive. The stable reversible-coloured photochromic additive having an SPF of at least 3 may be an added advantage.

The present invention also relates to a process for the preparation of the stable reversible-coloured photochromic additive, which is consistent in performance.

The present invention also relates to the use of the stable reversible-coloured photochromic additive in various fields including cosmetics, personal care, healthcare, paint, ink, textile, glass, optical lenses, plastic, articles and the likes.

### BACKGROUND OF THE INVENTION:

Stable and safe cosmetics with varying colourations are being continually explored and have become an important aspect to expand worldwide cosmetics business.

Cosmetics are an amazing tool to change or enhance one's appearance for various purposes including self-expression, identification, ritual, special events and advertising. These cosmetics may include skin products, including make-up and healthcare, hair products, paint and other types of products designed to alter the look of a person's face, amongst others. Recently, the evolution of colour changing make-up has driven the cosmetics industry to develop new cosmetic products with photochromic pigments.

The property of changing colour when one substance is irradiated with light and then returning to its original colour, when such irradiation is stopped, is referred to as photochromic. For example, this is used in many applications including cosmetics, healthcare, personal care, glass, textiles, inks, paints, toys, plastics, automotive, optical lenses, etc.

In the field of cosmetics, colour changing make-up, which changes colour using photochromic material, is known; particularly, it changes from its original colour when indoors to a changed colour when outdoors. Thus, photochromics change colour in response to sun light or black light. Photochromics are designed on the molecular level to interact with light or temperature or pH or moisture to maintain and evolve a desired colour shade. There are various materials, either organic or inorganic, which are used to impart a particular desired cosmetic look in a particular condition. For any effective photochromic material, the molecular structure must contain delocalized electrons in the outermost shells which enable strong absorption in the visible part of the spectrum and impart into a predictable cosmetic colour shade.

The photochromic material may be organic or inorganic. Many organic photochromic materials have delocalized electrons in the outermost shell, namely polycyclic aromatic hydrocarbons, coal tar products, azo dyes, quinolines, and the like. However, some of the organic photochromic materials are often carcinogenic and thus may be undesirable or unacceptable for use in the cosmetics or healthcare industry. These organic photochromic materials penetrate the skin barrier and thus may increase a risk of toxicity and threat of health hazard. Most importantly, the organic pigments deteriorate in the sunlight as the time passes. Therefore, the organic pigment is not a preferred option.

Inorganic photochromic materials, including metal oxides, are well known. An advantage of inorganic photochromic materials is that they are insoluble in water and lipids. The main advantage of the inorganic photochromic material, specifically non-nano material, is that it does not penetrate the skin barrier. Thus, non-nano inorganic photochromic material is safe. In contrast to organic pigments, inorganic pigments do not deteriorate in the sunlight. Among inorganic metal oxides, titanium dioxide and iron oxide are two most widely-used photochromic materials in the cosmetics industry.

Another class of metal oxides is transition metal oxides, including tungsten oxide which is a well-known photochromic material. Tungsten oxide (WO₃) is bright yellow in colour. When it is exposed to sun light, it changes its surface layer colour from bright yellow to dark blue. The colour change is due to transition from WO₃ to W₂O₅ in presence of blue-green light and IR light. But the reverse colour change to original colour is thermally driven or photo/light driven. It is used either alone or in combination with other metal oxides, including but not limited to titanium dioxide, aluminium oxide and silicone dioxide, as photochromic cosmetics.

Titanium oxide, or a mixture of titanium oxide and iron oxide coated mica pigment, has photochromic properties. Make-up consisting of the same illustrates darkening of the colour upon irradiation of light, where the intensity of light is strong, while it becomes the original colour in a room where the intensity of light is weak.

The cosmetic industry will keep on exploring for colour changing, particularly reversible colour changing, cosmetics with different shades. At the same time the cosmetics should be stable and consistent in their performance. The applicant is exploring to provide a stable reversible-coloured photochromic additive, which when used in cosmetic products, changes the colour to a desired colour shade, from pink to blue, when irradiated with outdoor sunlight and then returns to its original colour when indoors. It is a challenge to provide a stable reversible-coloured photochromic additive having enhanced strength of changed colour with stable and consistent performance when used in the cosmetic in the sunlight and with an SPF value of at least 3.

### OBJECTS OF THE INVENTION:

An object of the invention is to provide a stable reversible-coloured photochromic additive which changes colour from an original buff colour to a desired colour shade between pink to blue when exposed to sunlight and restores to the original colour in the absence of the sunlight and simultaneously having an SPF value of at least 3.

Another object of the invention is to provide a stable reversible-coloured photochromic additive which is safe and non-toxic.

Still another object of the invention is to provide a process for manufacturing the stable reversible-coloured photochromic additive of the present invention.

Yet another object of the invention is to provide a formulation comprising the stable reversible-coloured photochromic additive of the present invention, which is suitable for cosmetics, personal care, healthcare, paint, ink, textiles, plastics, glass, optical lenses, articles, automotive, toys and the like.

Yet another object of the invention is to provide a cosmetic or personal care or healthcare composition comprising the reversible-coloured photochromic additive of the present invention, which is safe, non-toxic, stable and consistent in performance and has an effective SPF value.

### SUMMARY OF THE INVENTION:

The invention provides a stable reversible-coloured photochromic additive comprising of titanium dioxide, psuedoboehmite, iron oxide hydroxide, tungsten oxide in a specific proportion. This additive changes colour from an original buff colour to a desired colour shade, between pink to blue, when exposed to sunlight and restores to the original colour in the absence of the sunlight and it may simultaneously have an SPF value of at least 3. The SPF value may be determined by COLIPA 2011.

The stable reversible-coloured photochromic additive may be safe and non-toxic, as the particle size is suitably at least 200 nm, which is non-nano. Particle size is calculated by measuring the particles using optical microscopy.

In the presently claimed invention, it is surprisingly found that the additive comprising titanium dioxide; psuedoboehmite; iron oxide hydroxide; and tungsten oxide, in the weight ratio of 89.02 to 99.97: 0.01 to 1: 4.99 to 0.01: 4.99 to 0.01, demonstrated colour change when exposed to sunlight and then restored to its original colour when indoors with enhanced intensity and stable performance. The proportion of the four components is very critical with respect to performance, including enhanced intensity or strength of colour, stability and consistent performance. Stability and consistent performance is very important for any cosmetic formulation. At the same time, the additive is non-toxic, being inorganic in nature as well as having non-nano particle size.

Accordingly, in a first aspect, the presently claimed invention is directed to a stable and consistent reversible-coloured photochromic additive, wherein the additive comprises titanium dioxide; psuedoboehmite; iron oxide hydroxide; and tungsten oxide in a ratio of 89.02 to 99.97: 0.01 to 1: 4.99 to 0.01: 4.99 to 0.01. This additive changes colour from an original buff colour to a desired colour shade, between pink to blue, when exposed to the sunlight and restores to the original colour in the absence of the sunlight.

Typically, the additive comprises titanium dioxide; psuedoboehmite; iron oxide hydroxide; and tungsten oxide in a ratio of 94.5 to 94.9: 0.1 to 0.5: 4.99 to 0.01: 4.99 to 0.01.

As noted above, the particle size is suitably at least 200 nm, which is non-nano. Particle size is calculated by measuring the particles using optical microscopy. Typically, the mean particle size of the additive is in the range of 200 to 500 nm. The mean particle size may be determined by ASTM E2651-13 (Particle size analysis).

Typically the additive has an SPF value of at least 3. The SPF value may be determined by COLIPA 2011.

It may be that the reversible-coloured photochromic additive is provided in non-calcined form. It may be used in this non-calcined form. Alternatively, and preferably, it may be calcined before use. It may also be ground, after calcining, before use. It will be appreciated that the product in non-calcined form is a useful precursor to the calcined product.

In one embodiment, the reversible-coloured photochromic additive is provided in calcined form.

In one embodiment, the reversible-coloured photochromic additive is a calcined component consisting of titanium dioxide, psuedoboehmite, iron oxide hydroxide, and tungsten oxide, in a weight ratio of 89.02 to 99.97 : 0.01 to 1: 4.99 to 0.01: 4.99 to 0.01.

In one embodiment, the reversible-coloured photochromic additive is provided in calcined and ground form. It may be that the reversible-coloured photochromic additive is ground to have a mean particle size of at least 200 nm, such as above 200 nm, or from 200 to 500 nm.

Accordingly, in a second aspect, the presently claimed invention is directed to a manufacturing process for making a reversible-coloured photochromic additive of the present invention, the process comprising the following steps:
(a) mixing titanium dioxide, psuedoboehmite, iron oxide hydroxide and tungsten oxide in a desired proportion, followed by jet milling to obtain a uniform mixture with a mean particle size of 200 to 500 nm;
(b) calcinating the mixture obtained in step (a) in a muffle furnace at a temperature in the range of 800 to 1000° C for at least 4 hours; and
(c) grinding the calcined product of step (b) by jet milling to get the reversible coloured photochromic additive with a mean particle size of at least above 200 nm.

It may be that the reversible coloured photochromic additive as obtained in step (c) has an SPF value of at least 3.

Typically, the proportion of titanium dioxide, psuedoboehmite, iron oxide hydroxide and tungsten oxide is in a ratio of 89.02 to 99.97: 0.01 to 1: 4.99 to 0.01: 4.99 to 0.01.

Typically, the mixture in step (b) is calcinated in a muffle furnace at a temperature in the range of 800 to 900° C.

Typically, the jet milling of each of steps (a) and (c) is carried out at a pressure of 1 to 8 bar and at a temperature from 5 to 15°C.

Typically, the jet milling of each of steps (a) and (c) is carried out at a pressure of 8 bar and at a temperature of 8°C.

In a third aspect, the presently claimed invention is directed to a formulation comprising the reversible-coloured photochromic additive of the first aspect of the invention. This may be manufactured according to the method of the second aspect of the invention. The formulation changes its colour in the presence of sun light and restores to the original colour in the absence of the sun light. The formulation may be used in a range of applications, including cosmetics, healthcare, personal care, ink, paint, textile, plastic, glass, optical lenses, toys, automotive or articles.

In a fourth aspect, the presently claimed invention is directed to a cosmetic or personal care or healthcare composition, for skin, hair or nail care, the composition comprising the reversible-coloured photochromic additive of the first aspect of the invention. This may be manufactured according to the method of the second aspect of the invention. The composition changes colour in the presence of sun light and restores to its original colour in the absence of the sun light, and may have an SPF value of at least 3.

Typically, the cosmetic or personal care or healthcare composition, for skin, hair or nail care, is in the form of a lotion, cream, gel, emulsion or powder.

In a fifth aspect, the presently claimed invention is directed to an article comprising the reversible-coloured photochromic additive of the first aspect of the invention. This may be manufactured according to the method of the second aspect of the invention. The article changes colour in the presence of sun light and restores to its original colour in the absence of the sun light, and may have an SPF value of at least 3.

The invention provides, inter alia, the subject matter of the following clauses:
1. A stable and consistent reversible coloured photochromic additive;
   the additive comprises titanium dioxide; psuedoboehmite; iron oxide hydroxide; and tungsten oxide in the ratio of 89.02 to 99.97 : 0.01 to 1: 4.99 to 0.01: 4.99 to 0.01;
   the additive changes colour from orginal buff to desired colour shade between pink to blue when exposed to sunlight and restores to original colour in the absence of the sunlight.
2. The additive of clause 1, wherein the additive comprises titanium dioxide; psuedoboehmite; iron oxide hydroxide; and tungsten oxide in the ratio of 94.5 to 94.9: 0.1 to 0.5: 4.99 to 0.01: 4.99 to 0.01.
3. The additive of clause 1, wherein the mean particle size of the additive is in the range of 200 to 500 nm.
4. The additive of clause 1, wherein the additive has synergistic SPF value of at least 3.
5. A manufacturing process for the reversible coloured photochromic additive of clause 1; the process comprises the following steps :
   (a) Mixing titanium dioxide, psuedoboehmite, iron oxide hydroxide and tungsten oxide in a desired proportion followed by jet milling to obtain a uniform mixture with a mean particle size of 200 to 500 nm;
   (b) Calcinating the mixture obtained in step (a) in a muffle furnace at a temperature in the range of 800 to 1000° C for at least 4 hours; and
   (c) Grinding the calcined product of step (b) by jet milling to get the reversible coloured photochromic additive with a mean particle size of at least above 200 nm and a synergistic SPF value of at least 3.
6. The process of clause 5, wherein the proportion of titanium dioxide, psuedoboehmite, iron oxide hydroxide and tungsten oxide is in the ratio of 89.02 to 99.97 : 0.01 to 1: 4.99 to 0.01: 4.99 to 0.01.
7. The process of clause 5, wherein the mixture of step (b) was calcinated in a muffle furnace at a temperature in the range of 800 to 900° C.
8. The process of clause 5, wherein the jet milling of step (a) and (c) is carried out at a pressure of 1 to 8 bar and at a temperature 5 to 15°C.
9. The process of clause 5, wherein the jet milling of step (a) and (c) is carried out at a pressure of 8 bar and at a temperature 8°C.
10. A formulation comprising the reversible coloured photochromic additive of clause 1 and manufactured according to clause 5; the formulation changes the colour in the presence of sun light and restore to the original colour in the absence of the sun light, including cosmetics, healthcare, personal care, ink, paint, textile, plastic, glass, optical lense, toys, automotive or article.
11. A cosmetic or personal care or healthcare for skin, hair or nail care comprising the reversible coloured photochromic additive of clause 1 and manufactured according to clause 5; the cosmetic or personal care or healthcare for skin, hair or nail care changes the colour in the presence of sun light and restore to the original colour in the absence of the sun light with SPF value of at least 3.

### DETAILED DESCRIPTION OF THE INVENTION:

The terms "a," "an," "the" and similar referents used in the context of describing the invention following claims are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Embodiments of the invention so claimed are inherently or expressly described and enabled herein.

As use herein, the term "photochromic" refers to a material, particularly inorganic material, which changes original colour to different colour shade or darkens when exposed to sunlight.

As use herein, the term "reversible photochromic" refers to a material, particularly inorganic material, which changes from its original colour to a different colour shade or darkens when outdoors and returns to the original colour when indoors.

As used herein, the term "nano" refers to a median primary particle size that is smaller than 100 nano meters (i.e. nm).

As used herein, the term "non-nano" refers to a median primary particle size that is bigger than 100 nm. Particle size is calculated by measuring the particles using optical microscopy.

As used herein, the term "weight percent (wt. %)" when used without qualification, typically refers to the weight percent of a particular solid component as compared with all solid components present, excluding medium or vehicle.

Unless stated otherwise, all numerical values relating to quantities or ratios are by weight.

### DETAILED DESCRIPTION OF THE EMBODIMENTS:

In the following description, the embodiments are described in sufficient detail to enable those skilled in the art to practice the invention. Other embodiments may be utilized, and structural, logical and other changes may be made without departing from the spirit and scope of the present invention. The following detailed description is, therefore, not to be taken in a limiting sense. The detailed description that follows begins with a definition section followed by a description of various embodiments of the invention. A series of examples are presented later followed by a brief conclusion.

Most modern cosmetics contain titanium dioxide (TiO₂) as well as iron oxide; particularly, nano particles of TiO₂ and iron oxide. These inorganic pigments blend more easily into the skin than that of their agglomerated form used earlier. These are known photochromic pigments as well as SPF additives. The cosmetic comprising the same shows a change in its darkness when exposed to sunlight. There is also growing demand from the consumer or fashion industry for colour changing cosmetics with new shades, in particular enhanced shades, when exposed to sunlight and having original shades when it is used indoors; simultaneously it should be safe and consistent in performance. The photochromic additive having acceptable SPF value of at least 3 will be an added advantage to the cosmetics industry. The reversible coloured photochromic additive may have a mean particle size of around 400 nm which makes the additive safe.

We have carried out the search and found that there is no additive present in the existing art which discloses the reversible-coloured photochromic additive of the present invention.

According to the present invention, there is provided a stable reversible-coloured photochromic additive which changes from an original buff colour to a desired colour shade, between pink to blue, when exposed to the sunlight and restores to the original colour in the absence of sunlight i.e. indoor, and simultaneously may have an SPF value of at least 3. It typically comprises titanium dioxide; psuedoboehmite; iron oxide hydroxide; and tungsten oxide in the ratio of 89.02 to 99.97: 0.01 to 1: 4.99 to 0.01: 4.99 to 0.01. Preferably, the additive comprises titanium dioxide; psuedoboehmite; iron oxide hydroxide; and tungsten oxide in the ratio of 94.5 to 94.9: 0.1 to 0.5: 4.99 to 0.01: 4.99 to 0.01.

The additive of the present invention suitably has a mean particle size of at least above 200 nm. In particular, the mean particle size of the additive is in the range of 200 to 500 nm; more particularly around 300 to 400 nm.

The relative amounts of the titanium dioxide, psuedoboehmite, iron oxide hydroxide, and tungsten oxide can be adjusted to achieve the desired changed colour shade, between pink to blue.

The additive of the present invention comprising more of the iron oxide hydroxide changes from its original colour to a desired colour shade between pink to red when exposed to sunlight and restores to its original colour in the absence of the sunlight. The additive simultaneously may have an SPF value of at least 3.

The additive of the present invention comprising more of the tungsten oxide changes from its original colour to a desired colour shade between violet to blue when exposed to sunlight and restores to its original colour in the absence of the sunlight. The additive simultaneously may have an SPF value of at least 3.

Similarly, the proportion of iron oxide hydroxide and tungsten oxide may be varied to achieve the desired colour shade, between pink to blue, in sunlight.

Psuedoboehmite, iron oxide hydroxide and tungesten oxide are required at particular concentrations to achieve the stable reversible-coloured photochromic additive of the present invention, to change from the original buff colour to desired colour shades with high intensity / strength in the presence of sunlight and to then restore to the original colour in absence of the sunlight. This also gives consistency in the performance to the additive of the present invention.

According to the present invention, there is also provided a manufacturing process for making a reversible-coloured photochromic additive of the present invention.

This process comprises the following steps:
(a) Titanium dioxide, psuedoboehmite, iron oxide hydroxide and tungsten oxide were admixed in a desired proportion and it was milled further by jet milling to obtain uniform mixture having a mean particle size of 200 to 500 nm;
(b) The mixture was calcinated in a muffle furnace at a temperature in the range of 800 to 1000° C for at least 4 hours; and
(c) The calcined product was ground by jet milling to get the reversible-coloured photochromic additive having a mean particle size of at least above 200 nm.

It may be that the reversible-coloured photochromic additive as obtained in step (c) has an SPF value of at least 3.

Typically, the proportion of titanium dioxide, psuedoboehmite, iron oxide hydroxide and tungsten oxide of step (a) may be varied based on the desired colour shades required in the sunlight.

Typically, the proportion of titanium dioxide, psuedoboehmite, iron oxide hydroxide and tungsten oxide is in the ratio of 89.02 to 99.97: 0.01 to 1: 4.99 to 0.01: 4.99 to 0.01. Preferably, the proportion is 94.5 to 94.9: 0.1 to 0.5: 4.99 to 0.01: 4.99 to 0.01.

The titanium dioxide used as starting material may optionally be anatase TiO₂. In one embodiment, the average particle size of the titanium dioxide starting material is 300 to 700 nm.

The psuedoboehmite (aluminium oxide-hydroxide) used as starting material may have an average particle size of 300 to 400 nm.

The iron oxide hydroxide used as starting material may have an average particle size of 300 to 400 nm.

The tungsten oxide used as starting material may have an average particle size of 300 to 400 nm.

Typically, the mixture in step (b) is calcinated in a muffle furnace at a temperature in the range of 800 to 900° C, more preferably at 840° C.

Typically, the jet milling of step (a) and (c) is carried out at a pressure of 1 to 8 bar and at a temperature of 5 to 15°C, particularly, the jet milling of step (a) and (c) may be carried out at a pressure of 8 bar and at a temperature of 8°C.

Typically, the mean particle size of the additive so obtained in the step (c) is of at least 200 nm; particularly in the range of 200 to 500 nm; more particularly at around 400 nm.

According to the present invention, there is provided a formulation comprising the reversible-coloured photochromic additive of the present invention. This formulation is suitable for many applications which require a change in the colour in the presence of sun light and restore to the original colour in the absence of the sun light, including cosmetics, healthcare, personal care, inks, paints, textiles, plastics, glass, optical lenses, toys, automotive, articles and the like.

According to the present invention, there is provided a cosmetic or personal care or healthcare composition, for skin, hair or nail care, comprising the reversible-coloured photochromic additive of the present invention and specifically used for skin, hair or nail care. Besides colour change, the cosmetic or personal care or healthcare composition may have an SPF value of at least 3. Preferably, the cosmetic or personal care or healthcare composition is in the form of a lotion, cream, gel, emulsion, or powder, depending on the bases used therein. Preferably, it is in the form of a cream, lotion or powder. It is suitably used in the sunscreen, sun-lotion, foundation, lip-stick, lip-gloss, lip balms, nail polish, hair colour, eye liner, eye shadow and pressed powder.

Typically, the cosmetic or personal care or health care compositions of the present invention may further comprise a UV filter agent or a topically active agent selected from an anti-tanning agent, an antimicrobial agent, a depigmentation agent, an anti-aging agent, antifungal agent, an insect repellent and a combination thereof.

The cosmetic or personal care or healthcare compositions of the present invention may further comprise additional additives like antioxidants, binders, biological additives, buffering agents, colourants, thickeners, polymers, astringents, fragrance, humectants, opacifying agents, conditioners, exfoliating agents, pH adjusters, preservatives, natural extracts, essential oils, skin sensates, skin soothing agents, skin healing agents, or the like.

An article comprising the reversible-coloured photochromic additive of the present invention is also provided.

Thus, the present invention facilitates the stable reversible-coloured photochromic additive which comprises titanium dioxide, psuedoboehmite, iron oxide hydroxide and tungsten oxide, where psuedoboehmite may act as booster and excites delocalised electron of titanium dioxide, iron oxide hydroxide and tungsten oxide in the presence of sun light from valence bond to covalent bond. This causes shift in the colour from original buff colour to any shade between pink to red and blue. When the sunlight exposure is removed, the excited electron again comes back to the valence bond. Thus, the presence of psuedoboehmite in the additive enhances the strength of the changed colour shades and the photochromic behaviour of the additive upon exposure to sunlight.

The median primary particle size of the additive of the present invention is suitably of at least 200 nm; that makes the additive safe and non-toxic to human as well as aquatic ecosystem.

The SPF value of the additive of the present invention is suitably of at least 3, which is an added advantage of the present invention.

The synergistic characteristic of the reversible-coloured photochromic additive of the present invention is useful in the cosmetics or personal care or healthcare or the formulations of glass, paint, automobile, toys, ink, textile, plastic, optical lenses or articles that require colour change at outdoors for the aesthetic cause or to improve look, with or without additional benefits of SPF.

### CHEMICALS USED

1. Titanium dioxide (Anatase) (TiO₂: 97-98%; Tinting Strength: 1220 min; Specific Gravity 3.8 - 3.9; Refractive Index 2.5; pH: 6.5 - 8; Bulk Density (tamped) : 0.7 ± 0.2 gm/cm³, Moisture: 0.4% max.; Oil Absorption: 20 - 25 gm / 100 gm; and Average Particle Size: 300 to 700 nm)
2. Aluminium oxide-hydroxide (CAS No: 1333-84-2: pore volume of 0.8 to 1.0 g/ml and minimum surface area of 250 m²/g, particle size of 300 to 400 nm)
3. Iron oxide hydroxide (particle size of 300 to 400 nm )
4. Tungsten oxide (particle size of 300 to 400 nm)

### EXAMPLES

We have exemplified the formulae of the stable reversible coloured photochromic additive of the invention in Table 1.

**Table 1. The stable reversible-coloured photochromic additive (i.e. RCPA) Formulae:**

| **RCPA** | **RCPA** | **RCPA** | **RCPA** | **RCPA** | **RCPA** | **RCPA** | **RCPA** | **RCPA** | **RCPA** |
|---|---|---|---|---|---|---|---|---|---|
| **→** | **(1)** | **(2)** | **(3)** | **(4)** | **(5)** | **(6)** | **(7)** | **(8)** | **(9)** |
| **Components** | | | | | | | | | |
| ↓ | | | | | | | | | |
| **Titanium dioxide (Anatase) (A) %** | **94** | **95** | **94.5** | **94** | **95** | **94.5** | **94.9** | **97** | **97** |
| **Aluminium oxide-hydroxide (B) %** | **1** | **0** | **0.5** | **1** | **0** | **0.5** | **0.1** | **0.1** | **0.1** |
| **Iron oxide hydroxide (C) %** | **4.99** | **4.99** | **4.99** | **0.01** | **0.01** | **0.01** | **4.99** | **2.9** | **0.01** |
| **Tungsten oxide (D) %** | **0.01** | **0.01** | **0.01** | **4.99** | **4.99** | **4.99** | **0.01** | **0.01** | **2.9** |

### Process:

1. The components A, B, C and D were mixed in various proportions as given in Table 1 by jet milling at 8 bar and maintaining temperature at 8° C;
2. The mixtures were calcined at 840°C for 4 hours;
3. The calcined products i.e. additives were cooled and further jet milled at 8 bar and maintaining temperature at 8° C to obtain additives (1) to (9).

The additives (1) to (9) were first tested for their SPF value by COLIPA 2011 and mean particle size by ASTM E2651-13 (Particle size analysis). The results of SPF and particle size analysis are illustrated in Table 2.

**Table 2. Test Results of the Additives (1) to (9) for SPF and mean particle size:**

| **RCPA** | **SPF Value** | **Mean particle size** |
|---|---|---|
| RCPA (1) | 3 | 400 nm |
| RCPA (2) | 3 | 400 nm |
| RCPA (3) | 3 | 400 nm |
| RCPA (4) | 3 | 400 nm |
| RCPA (5) | 3 | 400 nm |
| RCPA (6) | 3 | 400 nm |
| RCPA (7) | 3 | 400 nm |
| RCPA (8) | 3 | 400 nm |
| RCPA (9) | 3 | 400 nm |

CIELAB color space (1976) was used to measure colour and its strength. It expresses color as three values: L* for the lightness from black (0) to white (100), a* from green (-) to red (+), and b* from blue (-) to yellow (+). CIELAB was designed so that the same amount of numerical change in these values corresponds to roughly the same amount of visually perceived change. The colour of the additives (1) to (9) without exposing sunlight was measured and used as standard for comparison purpose. The additives (1) to (9) were then exposed to sunlight, leading to colour change. The change in the colour and its intensity was measured for each additive and compared with standards. The values of L*, a* and b* are illustrated in Table 3.

**Table 3: Results of CIELAB:**

| RCPA | (1) | (2) | (3) | (4) | (5) | (6) | (7) | (8) | (9) |
|---|---|---|---|---|---|---|---|---|---|
| a* | 0.426 | 0.116 | 0.777 | -0.112 | -0.233 | 0.439 | 0.6 | 0.16 | 0.036 |
| b* | -0.567 | -0.521 | 0.184 | -0.465 | -0.334 | -0.777 | -0.51 | 0.17 | -0. 51 |
| L* | -0.176 | -0.63 | 0.05 | -0.116 | -0.034 | -0.286 | -0.07 | 0.04 | -0.17 |
| Intensity | 105.213 | 103.3 | 120.8 | 117.86 | 117.337 | 137.96 | 99.97 | 99.2 | 139.61 |

According to Table 3, the additive having iron oxide in the range of 0.01 to 5 % changes colour from its original buff colour to a range of shades - from pink to red - at different concentrations, which was reflecting photochromic behaviour. At the same time, the intensity of changed colour was maximum at 0.5 % of psuedoboehmite and more reddish with 0.01 % of tungsten oxide. Similarly, in the case of the additive having tungsten oxide in the range of 0.01 to 5 %, this changes colour from its original buff colour to blue shades at different concentrations, which was reflecting photochromic behaviour. At the same time, the intensity of changed colour was maximum at 0.1 to 0.5 % of psuedoboehmite and was more blue with 0.01 % of iron oxide.

These results prove the synergistic property of the additives, with intense change in the colour shade and thus better photochromic behaviour than the individual components. The performance was found to be stable and consistent. The additive also showed SPF of 3 which was an added advantage.

All additives reflect a change of colour in presence of sunlight and restore to their original colour when indoors.

Thus, the additive of the presently claimed invention was safe and non-toxic to humans, as well as aquatic eco-system, as it was non-nano. It was converted into a cosmetic composition. It was useful in end applications including lotion, cream, foundation, lip-stick, nail polish and pressed powder.

The present invention has been described with reference to preferred embodiments, purely for the sake of understanding and not by way of any limitation and the present invention includes all legitimate developments within the scope of what has been described hereinbefore and claimed in the appended claims.

## Claims

1. A reversible-coloured photochromic additive, wherein the additive comprises titanium dioxide, psuedoboehmite, iron oxide hydroxide, and tungsten oxide, in a weight ratio of 89.02 to 99.97 : 0.01 to 1: 4.99 to 0.01: 4.99 to 0.01.

2. The additive as claimed in claim 1, wherein the additive comprises titanium dioxide, psuedoboehmite, iron oxide hydroxide, and tungsten oxide, in a weight ratio of 94.5 to 94.9: 0.1 to 0.5: 4.99 to 0.01: 4.99 to 0.01.

3. The additive as claimed in claim 1 or claim 2, wherein the mean particle size of the additive is in the range of from 200 to 500 nm.

4. The additive as claimed in any one of claims 1 to 3, wherein the additive has an SPF value of at least 3.

5. The additive as claimed in any one of claims 1 to 4, wherein the additive is provided in calcined form.

6. The additive as claimed in claim 5, wherein the additive is a calcined component consisting of titanium dioxide, psuedoboehmite, iron oxide hydroxide, and tungsten oxide, in a weight ratio of 89.02 to 99.97 : 0.01 to 1: 4.99 to 0.01: 4.99 to 0.01.

7. A manufacturing process for obtaining a reversible-coloured photochromic additive as defined in claim 5 or claim 6, wherein the process comprises the following steps:
(d) mixing titanium dioxide, psuedoboehmite, iron oxide hydroxide and tungsten oxide, followed by jet milling to obtain a uniform mixture with a mean particle size of from 200 to 500 nm;
(e) calcinating the mixture obtained in step (a) in a muffle furnace at a temperature in the range of from 800 to 1000° C for at least 4 hours; and
(f) grinding the calcined product of step (b) by jet milling, to obtain a mean particle size of at least 200 nm.

8. The process as claimed in claim 7, wherein the proportion of titanium dioxide, psuedoboehmite, iron oxide hydroxide and tungsten oxide as mixed in step (a) is in the ratio of 89.02 to 99.97 : 0.01 to 1: 4.99 to 0.01: 4.99 to 0.01.

9. The process as claimed in claim 7 or claim 8, wherein in step (b) the mixture is calcinated in a muffle furnace at a temperature in the range of from 800 to 900°C.

10. The process as claimed in any one of claims 7 to 9, wherein the jet milling of each of step (a) and step (c) is carried out at a pressure of from 1 to 8 bar and at a temperature of from 5 to 15°C.

11. The process as claimed in claim 10, wherein the jet milling of each of step (a) and step (c) is carried out at a pressure of 8 bar and at a temperature of 8°C.

12. A formulation or an article comprising the reversible coloured photochromic additive as defined in any one of claims 1 to 6 and/or as manufactured by the process of any one of claims 7 to 11.

13. The formulation as claimed in claim 12, wherein the formulation is for use in cosmetics, healthcare, personal care, inks, paints, textiles, plastics, glass, optical lenses, toys, automotive or articles.

14. The formulation or article as claimed in claim 12, wherein the formulation or article is a cosmetic or personal care or healthcare composition, for skin, hair or nail care.

15. The formulation or article as claimed in claim 14, which is in the form of a lotion, cream, gel, emulsion or powder.
